(19) Europäisches Patentamt
European Patent Office
Office européen des brevets

(11) Publication number : 0 327 163 B1

(12) # EUROPEAN PATENT SPECIFICATION

(45) Date of publication of patent specification : 12.04.95 Bulletin 95/15

(51) Int. Cl.$^6$ : **G01N 33/26**, G01N 33/53, G01N 33/94, G01N 33/14

(21) Application number : 89200164.5

(22) Date of filing : 25.01.89

(54) **Detection of chemicals by immunoassay.**

(30) Priority : 02.02.88 GB 8802237

(43) Date of publication of application : 09.08.89 Bulletin 89/32

(45) Publication of the grant of the patent : 12.04.95 Bulletin 95/15

(84) Designated Contracting States : AT BE CH DE ES FR GB GR IT LI LU NL SE

(56) References cited :
EP-A- 0 260 829
WO-A-88/09798
DE-A- 2 410 033
J. AGRIC. FOOD CHEM., vol. 33, 1985; M.M. KELLEY etal., pp. 962-965
J. AGRIC. FOOD CHEM., vol. 30, 1982, American Chemical Society; S.I. WIE et al, pp. 949-957;

(73) Proprietor : BIOCODE, INC.
P.O. Box 648
275 Mill Way
Barnstable, MA 02630 (US)

(72) Inventor : Wraith, Michael John
3 Berkeley Court
Sittingbourne Kent (GB)
Inventor : Britton, David William
36 Bysingwood Road
Faversham Kent (GB)

(74) Representative : Nicholls, Kathryn Margaret et al
MEWBURN ELLIS
York House
23 Kingsway
London WC2B 6HP (GB)

EP 0 327 163 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method of detection of chemical marker compounds by immunoassay and an assay kit enabling such detection to be carried out in the field. The invention is particularly, though not exclusively, directed towards the detection of marker compounds in liquid products, especially in oil based products such as lubricating oils.

A major problem experienced in many areas of the world and in connection with many different products is that of product counterfeiting.

Throughout the world, traders provide the materials they sell with a visually distinctive appearance in order that customers can distinguish their materials from those of others. As a result, their customers learn to associate the visually distinctive appearance of the material with certain standards of quality, and, if they are satisfied with those standards, will buy materials provided with that visually distinctive appearance in preference to others.

Once customers have acquired a preference for materials provided with a particular visually distinctive appearance,the traders become vulnerable to product counterfeiting.

A counterfeit product consists of material that is provided with a visually distinctive appearance confusingly similar to that with which material of a genuine product has been provided. Customers seeing the visually distinctive appearance provided to the counterfeit material buy this material in the expectation that they are buying a genuine product.

There are many ways known of providing materials with a visually distinctive appearance. In general, the visually distinctive appearance is provided either directly to the material or to an article with which the material is associated, for example a label, wrapper or container. The visually distinctive appearance may be, for example, a distinctive shape or configuration, a distinctive marking, or a combination of the two. A particularly preferred visually distinctive appearance is a trade mark.

The material of a counterfeit product may be the same as, or different from the material of a genuine product. Often the material of the counterfeit product is the same, but of inferior quality.

There are many ways in which product counterfeiting can take place. In one way, the product counterfeitor provides his counterfeit material with a visually distinctive appearance copied from that of a genuine product. In another way, the product counterfeitor adulterates or replaces the material of a genuine product without affecting the visually distinctive appearance with which the material of the genuine product had been provided.

An example of such a problem lies in the adulteration of lubricating oils, or other oil based products, by addition of a counterfeitor's oil to a genuine product. Such adulteration is not only financially damaging to the oil manufacturer but the consequent lowering of performance which can occur can cause damage to the consumer and consequently harm the reputation of the genuine product.

A method of overcoming this problem has been previously proposed involving the incorporation of dye in the product. Such a strategy is easily copied. However if a marker is incorporated which is not readily detectable by visual means, samples of product which may have been adulterated may need to be returned to a base laboratory for physical analysis, for example by chromatographic techniques, before one can determine the extent, if any, of adulteration. The consequent delay is inconvenient for, for example, a distributor in an area where there are no readily available back-up laboratory facilities, and lowers the effectiveness of the technique in deterring counterfeitors.

There is clearly a need for a method which can be used in the field for detecting attempts at product counterfeiting.

European patent application publication number EP-A-0260829 discloses monoclonal and polyclonal antibodies which may be used to identify chlorinated phenols, particularly pentachlorophenol, in materials and to determine the concentration of the chemical in those materials. It is noted in the introduction to the specification that pentachlorophenol is added to materials as a pesticide or a preservative. However, EP-A-0260829 does not disclose the identification of chlorinated phenols in products, that is to say materials provided with a visually distinctive appearance. Furthermore, EP-A-0260829 does not disclose the use of chlorinated phenols as marker compounds. In particular, EP-A-0260829 does not disclose associating a chlorinated phenol with a genuine product for the purpose of distinguishing it from a counterfeit product.

In a poster presentation given by M.J. Wraith et al at the 6th International Congress of Pesticide Chemistry, August 10-15th, 1986 in Ottawa, Canada entitled Development of Immunoassay Methods for Pyrethroid Insecticides, there were disclosed protein conjugates of m-phenoxybenzoic acid and dichlorovinyl cyclopropane carboxylic acid, and polyclonal antibodies prepared using these protein conjugates. Also disclosed was the analysis of the cypermethrin metabolites, m-phenoxybenzoic acid and dichlorovinyl cyclopropane carboxylic acid in black tea, water and soil. However, no disclosure was made of the use of either m-phenoxybenzoic acid or dichlorovinyl cyclopropane carboxylic acid as a marker compound, nor of the detection by immunoassay of

either compound in any material provided with a visually distinctive appearance.

According to this invention we provide a method of authenticating a product comprising:

associating a marker compound with the product thereby to produce a "genuine" product, said marker compound, when associated with said product being visually undetectable; subsequently

profiding from a test product whose authenticity it is desired to determine a sample, dissolved in aqueous medium, of any said marker compound associated with said test product;

identifying any said marker compound in said sample by an immunoassay specific for said marker; and

comparing the result of the immunoassay carried out on the sample from the test product with that obtainable when the same immunoassay is carried out on a sample in aqueous medium provided from said "genuine" product.

It will be appreciated that the marker compound may be associated with the product in a wide variety of ways. Thus the marker compound may be present in or on all or part of the product, or in or on all or part of a label, wrapper or container associated with the product. The marker compound is usually mixed with the product, but may alternatively be present independently of the product, for example the marker may be present in the product packaging or labelling.

The product may be solid or fluid.

Examples of solid products include pharmaceutical tablets, capsules and powders; solid formulations of agrochemicals such as insecticides, herbicides, fungicides and fertilisers; textiles such as clothing; recordings such as gramophone records, tape cassettes, floppy discs and compact discs; electrical goods such as television sets, computers and radios; motor vehicle components and cameras.

Examples of fluid products include oil-based products such as lubricating oils, gasoline, diesel and liquified petroleum products; paints; perfumes; cosmetics; drinks such as wine, whisky, sherry, gin and vodka; liquid pharmaceutical formulations such as syrups, emulsions and suspensions; liquid agrochemical formulations; and industrial solvents. The product is preferably liquid, preferably an oil based product such as a lubricating oil.

The marker compound should be visually undetectable and substantially water soluble. It will be appreciated that the marker compound should also be capable of being detected by immunoassay, and be compatible with the product which it marks. Preferably it will be non-toxic. One skilled in the art of immunoassay technology would have no difficulty in identifying compounds suitable for use as marker compounds.

Usually compounds suitable for use as marker compounds will be organic compounds comprising carbon and hydrogen atoms and one or more heteroatoms selected from oxygen and nitrogen, and salts thereof. Optionally one or more other heteroatoms may also be present, for example halogen atoms such as chlorine, bromine or iodine atoms, phosphorus atoms or sulphur atoms. Preferred compounds are carboxylic acids, carboxylic acid esters, ketones, alcohols, phenols, amines, anilines, nitriles and ethers, and salts thereof. Particularly preferred compounds are aromatic carboxylic acids, e.g. benzoic acids; phenols; ethers of polyhydric alcohols and phenols; amino acids; peptides; proteins; lipids; carbohydrates, e.g. sugars and polysaccharides; nucleic acids; and polynucleic acids, e.g. deoxyribonucleic acids and ribonucleic acids.

When the product is an oil-based product such as a lubricating oil, the compound used as a marker compound preferably has a log P in the range of from -2.5 to + 5.0, preferably from -1.5 to 4.5, most preferably from 0 to 4.0. (Log P as used herein means the logarithm of the partition coefficient of a compound between octanol and water at 25°C). Thus, m-phenoxybenzoic acid, for example, has a log P of 3.9.

It is possible to produce antibodies which are selective for a particular optically active form of a compound. Since it is difficult to distinguish between optically active forms of compounds by conventional analytical techniques, particularly when only trace quantities of the compound are available for analysis, the use of optically active marker compounds may be particularly advantageous.

A compound suitable for use as a marker compound has been found to be m-phenoxybenzoic acid, but it will be appreciated that a wide range of compounds are suitable for such a purpose so long as they are compatible with and non-deleterious to the product being marked. Thus the use of oil-compatible, water-compatible and solids - compatible compounds as marker compounds is envisaged dependent on the product being marked.

When the product is a liquid, the marker compound is preferably colorless and soluble in the liquid product so that its presence can only be detected by subsequent assay. It is preferably also odourless.

Preferably only trace quantities of marker are used. Typically a marker compound will be incorporated with a product at a concentration in the range of from 1 part per billion (ppb) to 25 parts per million (ppm). Preferably the concentration will be in the range of from 100ppb to 15ppm, more preferably 1ppm to 10ppm. Thus, for example, the concentration of marker compound may be up to about 10ppm, and dependent on the marker compound, even a few ppb may be sufficient for detection.

The ability to detect concentrations of marker compound at 25 ppm or below is a particular advantage of

the method according to the invention. Thus only small quantities of marker compound need to be used.

According to another aspect, the invention provides an oil-based product comprising from 1ppb to 25ppm of a visually undetectable, substantially water-soluble marker compound having a log P in the range of from -2.5 to 5.0.

If the marker compound is incorporated with the product in an aqueous medium, the immunoassay may be carried out directly on a sample thereof, if necessary after filtration to remove solids. Otherwise the marker compound must be brought into aqueous solution.

In general providing a sample of marker compound in aqueous solution will comprise one or more steps selected from solvent extraction of the marker compound from the product; dilution of the product with an aqueous solvent; filtration; evaporation; and solid phase extraction of the marker compound, e.g. purification of the marker compound using an ion exchange resin or chromatography, for example using silica. In the case of a marked oil-based product solvent extraction appears to be necessary.

The solvent chosen for extracting the marker compound from the product prior to assay naturally depends on the natures of the product and the marker. Depending upon the natures of the product and the marker, the solvent will in general comprise one or more of water; hydrocarbons, for example benzene, toluene, xylene, hexane, heptane and octane; sulphoxides, for example dimethylsulphoxide; halogenated hydrocarbons; for example chlorobenzene, methylene chloride, chloroform and carbon tetrachloride; ethers, for example diethyl ether, dioxan and tetrahydrofuran; amides, for example dimethylformamide and dimethylacetamide; nitriles, for example acetonitrile; alcohols, for example methanol, ethanol and propanol; esters, for example ethyl acetate; and ketones, for example acetone. Preferably the solvent comprises water and/or a water miscible organic solvent. When testing lubricating oils marked with m-phenoxybenzoic acid, a suitable extracting solvent is a mixture of a diluent for the oil such as hexane, a water-miscible organic solvent such as acetonitrile, and water. Optionally the extraction solvent may also comprise buffer salts such as Tris buffer (Tris[hydroxymethyl]aminomethane). The solvent system used preferably yields the extracted marker compound in an aqueous phase suitable directly for the subsequent immunoassay.

The immunoassay is carried out using a pre-prepared antibody to the chosen marker compound. Such an antibody is raised by known techniques which allow one to obtain monoclonal or polyclonal antibodies specific to a particular compound. Preferably monoclonal antibodies are used.

Antibodies are proteins produced in animals by antibody-producing cells known as B-lymphocytes in response to the exposure of the animal to foreign compounds (antigens). These antibodies bind specifically to the particular compound which stimulated their production.

Antibody-producing cells arise in the spleen of an animal when the animal has been immunised with an immunogenic compound. Not all compounds are immunogenic. In general, compounds with a molecular weight of below 2,000 are not immunogenic. However, antibodies which are specific for such compounds (known as haptens) may be obtained by chemically binding the hapten to a larger immunogenic carrier such as a carbohydrate or protein, and immunising an animal with the resultant immunogenic conjugate.

The attachment of hapten to immunogenic carrier may be achieved using a bifunctional molecule in a two-stage chemical reaction. This provides a spacer arm between the hapten and carrier which may improve the immune response. In order for the compound to be chemically linked to the carrier or bifunctional molecule, it should itself contain a functional group. Preferred functional groups are amino groups, hydroxyl groups and carboxyl groups.

When an animal has been immunised with an immunogenic substance, a wide variety of different antibody-producing cells are stimulated. The antibodies produced by such a response are known as polyclonal antibodies.

Polyclonal antibodies raised against a particular compound do not all bind with the same specificity to that compound. However, it is possible to obtain antibodies which all bind with the same specificity and affinity to a compound. These antibodies are known as monoclonal antibodies.

In order to obtain such monoclonal antibodies, antibody-producing cells are firstly extracted from the spleen of an immunised animal. These cells are then fused with myeloma cells to produce hydridomas. Fusion may be achieved, for example, by treatment with polyethylene glycol. The hybridomas are capable of producing antibodies, like the precursor antibody-producing cells, but are immortal; they are capable of continuous growth in vitro. A number of myeloma cells suitable for fusing with antibody producing cells are known and readily available to those skilled in the art. An example of a suitable myeloma cell which is readily available is PX3-63-AG8-653. This cell is available, for example, from the American Type Culture Collection, Rockville, Maryland, USA under the number ATCC CRL 1580.

Once the antibody-producing cells and the myeloma cells have been fused, the resultant hybridoma cells are separated from the unfused cells and cloned by repeated limiting dilution. Cloned hybridomas are then tested to determine which are producing the desired antibodies. This testing may be achieved, for example,

by competitive enzyme linked immunosorbent assay (ELISA). Specificity and affinity for a compound may be assessed by the addition of free compound to the ELISA test system to evaluate the ability of the free compound to inhibit binding of the monoclonal antibody to compound which is bound to a solid phase.

Once a particular hybridoma has been selected, monoclonal antibodies may readily be produced in large quantities using well known techniques. If desired, these antibodies may be labelled with an enzyme; e.g. horse radish peroxidase or alkaline phosphatase.

Techniques for producing polyclonal and monoclonal antibodies for a compound are well known to those skilled in the art. Examples of references in which such techniques are described include Methods of Enzymology Volume 70 and Volume 73 Immunochemical Techniques parts A and B respectively Edited by Van Vunakis, H and Langone, J.L., Published by Academic Press 1980 (Part A) and 1981 (Part B), and Kohler, G. and Milstein, C, Nature, Vol 265, p 495 (1975).

According to another aspect the invention includes novel monoclonal antibodies to m-phenoxybenzoic acid.

The method according to the invention may comprise identifying the marker compound in the sample qualitatively or quantitatively. The method preferably comprises identifying the marker compound quantitatively. In this way, the degree of adulteration may be ascertained.

Assay of the marker compound by contact with the antibody is preferably accomplished by competitive enzyme-linked immunosorbent assay (ELISA), although other immunoassay methods may be employed, including enzyme-mediated immunoassay and sandwich immunometric assays. Actual detection of the result of the assay may be by colorimetric means or by alternative detection means such as chemiluminescence or fluorescence.

It will be appreciated that such a detection method is well suited to field operation as no complex laboratory equipment is required. Thus, in order to carry out the present invention, there may be provided an assay kit for detecting the presence of a visually undetectable, substantially water soluble marker compound associated with a product, the kit comprising means for providing a sample of said marker in aqueous medium, immunoassay means including antibodies specific for the marker compound, detection means for monitoring the immunoassay and means for comparing the result of the immunoassay with the result expected from a genuine product.

A means for providing a sample of said marker in aqueous medium may comprise any solvent necessary for bringing the marker into aqueous solution and/or filtration means to remove unwanted solids and/or solid phase extraction columns (for example columns containing an ion exchange resin or a chromatography medium such as silica).

An immunoassay means may comprise monoclonal or polyclonal antibodies, supplied in an appropriate quantity. It may also comprise a hapten, which may be bound to a solid phase.

A detection means for monitoring the result of the immunoassay may be, for example, means to produce and/or measure a colour reaction. Thus a detection means may comprise an enzyme and a substrate for the enzyme. The enzyme may be attached to the hapten, the anti-hapten antibody, or a second antibody directed against the anti-hapten antibody. Examples of enzymes include horse radish peroxidase and alkaline phosphatase. Examples of substrates include o-phenylenediamine dihydrochloride; Amerlite Signal Reagent (available from Amersham International PLC); and p-nitrophenol phosphate. It will be appreciated that an external detection device such as a spectrophotometer, luminometer of fluorometer may be employed. In this way not only the existence of the marker compound but also the amount present can be determined, thus giving an indication of the extent of adulteration of the product.

A means for comparing the result of the immunoassay with that expected from a genuine product may comprise instructions describing the result expected of a genuine product (comprising, for example, a colour chart, calibration table or calibration curve), or it may comprise a sample of marked material identical to marked genuine product (to be analysed alongside the unknown sample).

The kit is preferably provided with a representation of the visually distinctive appearance provided to the material of the genuine product. For example the kit may be provided with a representation of a trade mark with which the material of the genuine product is provided.

The ability to provide assay means in kit form ensures that a man in the field, such as a distributor of a product in an environment distant from the product source can quickly check the authenticity of the product without recourse to laboratory facilities.

The invention will now be further described with reference to the following examples.

Example 1

Preparation of Protein Conjugates of Compound intended for use as a marker compounds

A series of protein conjugates of m-phenoxybenzoic acid (a hapten, referred to hereinafter as 'PBA') were prepared by firstly preparing a suitable reactive derivative from PBA by chemical synthesis followed by conjugation of the derivative with the protein. The derivatives were prepared with a $^{14}C$ radio label to allow monitoring of the subsequent protein conjugate to check for removal of reagents and to calculate the loading of the protein with the hapten.

i) Preparation of PBA derivatives

m-Phenoxybenzoic acid was reacted with thionyl chloride in benzene to give the corresponding benzoyl chloride which was subsequently reacted with 4-aminobutyric acid in the presence of sodium hydroxide followed by acid hydrolysis to give a derivative a) of formula I

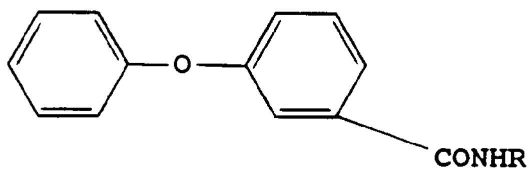

(I)

where R is $-(CH_2)_3COOH$.

Two further derivatives were prepared by reacting the intermediate benzoyl chloride with b) glycine and c) glycyl glycine in the presence of sodium hydroxide followed by acid hydrolysis to give b) a derivative of formula I where R is $-CH_2COOH$ and c) a derivative of formula I where R is $-CH_2CONHCH_2COOH$.

The derivative a) of formula I was also prepared by reacting benzyl 4-aminobutyrate and 3-(3′-dimethylaminopropyl)-1-ethyl carbodiimide in aqueous tetrahydrofuran to give a compound of formula I where R is $-(CH_2)_3COOCH_2Ph$ followed by hydrogenolysis with a palladium on charcoal catalyst in tetrahydrofuran to yield derivative a).

The derivatives were converted to their sodium salts (which dissolved readily in water) by addition of stoichiometric amounts of sodium bicarbonate or carbonate in water and tetrahydrofuran added until a homogenous solution was obtained followed by evaporation to dryness.

ii) Preparation of Protein Conjugates

The derivatives prepared as described in i) above, in the form of their sodium salts, were each dissolved in water, adjusted to pH 8 and cooled to 0°C. A solution, also cooled to 0°C, of 3-(3′-dimethylaminopropyl)-1-ethyl carbodiimide was added to the derivative sodium salt and allowed to stand for 2 minutes to complete formation of the isourea carboxylate.

Each of derivatives was then bonded to one of the following proteins dissolved in distilled, deionised water and filtered:

bovine serum albumin (M.W. about 68,000)
chicken gamma globulin (M.W. 125,000 to 750,000)
keyhole limpet hemocyanin (M.W. 3,000,000 to 7,000,000)

The loading was carried out by adding the solution over one minute to the protein with stirring and the mixture kept at 5°C for several hours to complete the bonding. pH was adjusted to pH 8 as necessary. The loaded protein was dialysed with saline phosphate buffer pH 7.3 for from 5 to 7 days with daily changing of dialysate and the loading determined by $^{14}C$ radioactivity measurement.

Example 2

Production of Monoclonal Antibodies

A conjugate of PBA and bovine serum albumin, 15 moles PBA per mole of protein, was used to produce the antibodies. The PBA-bovine serum albumin was prepared using derivative a) of Example 1 i) in the procedure of Example 1 ii).

Six mice (Balb/c, female) were immunised subcutaneously with a 1:1 emulsion of complete Freund's adjuvant and PBA conjugate (0.1ml, 50 µg). Each animal received a further 3 injections at 3 weekly intervals but with incomplete adjuvant. Under a similar regimen, a further six animals received a higher dose of conjugate (200µg). Serum samples from the twelve animals were tested for specific binding to PBA by means of an enzyme-linked immunosorbent assay (ELISA).

The spleen was removed from the animal producing the highest serum concentration of antibodies and the splenocytes used in a fusion with PX3-63-AG8-653 myeloma cells (available from the American Type Culture Collection, Rockville, Maryland, USA, under the number ATCC CRL 1580). Hybridoma cells were distributed into ten 96-well microtitre plates. Following cell growth, supernatant tissue culture fluids were tested by ELISA for antibody production. Specificity was assessed by the addition of free PBA to the test system to determine the inhibition of binding of the antibodies to the solid phase PBA target in the ELISA method. Cells from several positive wells were grown to produce cell stocks and then cloned by the limiting dilution technique. Following further cell growth the resulting supernatants were tested as above and the contents of several positive wells grown-up and cloned again. The cells in wells identified as positive, following the second cloning, were grown-up to produce supernatants containing sufficient monoclonal antibodies for preliminary assay development.

In order to generate sufficient monoclonal antibodies for medium-term requirements, 5 clonal cell hybridomas were chosen for the production of antibody-rich ascites fluid. Ten, pristane-primed, female Balb/c mice per hybridoma, were each inoculated intraperitoneally with up to $10^7$ hybridoma cells. Ascites fluids were harvested, pooled and stored deep frozen.

Antibodies were also prepared by growing hybridoma cell in vitro in stirred tissue culture vessels.

A sample of one of the clonal cell hybridomas has been deposited with the European Collection of Animal Cell Cultures (ECACC), PHLS Centre for Applied Microbiology and Research, Porton Down, Salisbury SP4 0JG, United Kingdom, with effect from 10th January, 1989 under accession number 89011001.

## Example 3

### Assay of a Marked Lubricating Oil

A marked lubricating oil (available under the Shell Trade Mark Rimula X) was prepared containing 10 ppm of m-phenoxybenzoic acid. A series of 2ml samples was prepared containing varying percentages of marked oil and unadulterated oil.

PBA was extracted for assay from each oil sample by the following method.

### Extraction of PBA from Oil

1. The oil sample on which the authenticity check is to be carried out was placed in a sealable vessel. Five volumes of hexane and 1 volume of 20% acetonitrile in 0.05M Tris/HCl pH 7.5 were then added to the oil and the vessel sealed. The mixture was then shaken for one minute and the resulting suspension allowed to separate. This took approximately 30 seconds.
2. An aliquot was taken from the lower phase of the separated mixture using a disposable plastics pipette and applied to a 3ml NH2 Bond Elut column (an ion exchange resin column obtained from Jones Chromatography). The sample of extract was passed through the column by the application of pressure and the column then washed with 4 x 1 ml aliquots of distilled water.
3. The column was then eluted with 2 ml of a solution of 0.05% (v/v) Tween 20 in saline. This solution removed any bound PBA. Each recovered PBA solution was then subjected to competitive enzyme linked immunosorbent assay (ELISA) by the following method.

### Immunoassay Method (with detection by colorimetric means)

1. A plastic well/tube was coated with a fixed level of a PBA-chicken gamma globulin conjugate prepared as described in Example 1. To achieve this coating, a measured aliquot of 100 µl conjugate, at a concentration of 10 µg/ml was placed in the well/tube. This was then incubated at a carefully controlled temperature for a fixed time and a reproducible level of coating was achieved by adsorption. After the coating period, the wells were washed and could be stored at 4°C.
2. The PBA-containing solution to be assayed was placed in a pre-coated well in the presence of a limiting level of specific monoclonal antibody prepared as described in Example 2. The sample of antibody was used at a dilution of 1:1000. The basis of the assay is the competition for binding of this antibody between

the PBA in solution and PBA which is immobilized on the surface of the well as conjugate. After a fixed period of time the solution in the well was removed and the well washed. The antibody remaining in the well after washing is that which bound to the immobilized PBA, the level of antibody remaining is thus inversely proportional to the level of PBA which was previously present in free solution.

3. A solution of second antibody-enzyme conjugate was added to the well. The second antibody enzyme conjugate used was IgG (alkaline phosphatase linked to rabbit anti-mouse immunoglobulin G - available from ICN Biologicals) at 1:1000 dilution, 100 μl per well. This conjugate binds to any of the primary antibody which has remained, bound to the immobilized conjugate in the well. The second antibody-enzyme conjugate was added in excess and, again, unbound material was removed by washing. After washing, the level of second-antibody-enzyme conjugate remaining in the well is directly proportional to the level of primary antibody bound in step 2 described above.

4. A solution containing a substrate for the enzyme of the second antibody-enzyme conjugate was added to the well and the level of enzyme present was determined by measuring the formation of coloured product. The substrate was p-nitrophenyl phosphate disodium salt (available from Sigma Chemical Co. as Sigma 104 phosphatase substrate) at a concentration of 1 mg./ml in 10% diethanolamine buffer pH 9.8. The formation of yellow coloured product was measured at 405 nm using a vertical beam visible light absorbtion spectrophotometer (MR610 Microplate reader - Dynatech).

Two sets of results of the colorimetric assay of the oil samples containing varying percentages of marked oil are given in Table 1 below. The results are expressed as a percentage of marked oil in the sample.

<u>Table 1</u>

| Sample No. | 1 | 2 | 3 | 4 | 5 | 6 | 7 | 8 | 9 | 10 |
|---|---|---|---|---|---|---|---|---|---|---|
| Calculated | 75 | 25 | 100 | 0 | 50 | 0 | 33 | 100 | 10 | 50 |
| Observed Set A | 47 | 14 | 100 | 0 | 50 | 0 | 29 | 100 | 0 | 42 |
| Observed Set B | 76 | 33 | 93 | 0 | 55 | 0 | 22 | 118 | 31 | 55 |

<u>Example 4</u>

<u>Assembly of an Assay Kit and its use in an Assay of a Marked Lubricating Oil</u>

An assay kit, suitable for testing up to three possibly counterfeit samples of lubriating oil, was assembled. The kit comprised:-

(1) 5 plastic tubes coated with a PBA-chicken gamma globulin conjugate (prepared as described below);
(2) 5 ion exchange resin columns, (3ml NH2 Bond Elut columns, obtained from Jones Chromatography);
(3) 5 10 ml volumes of extracting solvent, consisting of 8ml hexane and 2ml of 40% acetonitrile in 0.05M Tris/HCl, pH 7.5;
(4) 1 sample of lubricating oil containing 10ppm of m-phenoxybenzoic acid (representing a sample of a marked genuine product);
(5) 1 sample of lubricating oil containing no m-phenoxybenzoic acid;
(6) 5 2ml volumes of PBA-Specific monoclonal antibody diluted ten-fold in phosphate buffered saline (PBS, available in tablet form from Oxoid) containing 0.05% w/v Tween 20 (polyoxyethylene-sorbitan monolaurate, available from Sigma Chemical Company, Catalogue Number P1379);
(7) 1 volume of antibody-enzyme conjugate (comprising horse radish peroxidase conjugated rabbit immunoglobulins to mouse immunoglobulins, available from DAKO Limited Catalogue Number P161);
(8) Chemiluminescent substrate (Amerlite Signal Reagent, available from Amersham International PLC, Catalogue Number LAN 4400);
(9) Wash solution, consisting of 0.05% (v/v) Tween 20 in saline (ST);
(10) Column wash, consisting of distilled water; and
(11) 1 instruction sheet.

<u>Preparation of plastic tubes coated with a PBA-chicken gamma globulin conjugate</u>

Plastic tubes were coated with a fixed level of a PBA-chicken gamma globulin conjugate prepared as described in Example 1. To achieve this coating, a measured aliquot of 500 µl conjugate, at a concentration of 20 µg/ml was placed in the tube. This was then incubated for 3 hours at room temperature and a reproducible level of coating was achieved by adsorption. After the coating period, the tubes were washed with the saline/Tween solution (ST), described in Example 3, dried and stored dessicated, preferably at 4°C, until required.

Use of Assay Kit to Assay Marked Lubricating Oils

1. In order to carry out the assay, the first step is to prepare the chemiluminescent substrate, following the manufacturer's instructions.
2. The two samples of lubricating oil belonging to the kit and three "unknown" samples of lubricating oil are extracted using the 5 volumes of extracting solvent and 5 ion exchange columns, according to the method of Example 3. The columns are then washed with two 2ml aliquots of column wash.
3. Each of the columns is then eluted with one 2ml aliquot of wash solution into small glass bottles containing the 2ml volumes of PBA-specific monoclonal antibody.
4. The bottle contents are then mixed, and at least 500 µl from each of the 5 mixtures is transferred to the five plastic tubes coated with PBA-chicken gamma globulin conjugate. After 5 minutes the solutions in the tubes are tipped away and the tubes washed once with wash solution.
5. 500 µl of a solution of the antibody-enzyme conjugate is added to each of the tubes. After a 5 minute incubation the contents of the tubes are tipped away and the tubes are washed 5 times with wash solution.
6. The chemiluminescent substrate, 1 ml, is then added to the tube and after 2 minutes the light output measured, following the manufacturers instructions for use of a portable, battery powered, tube luminometer (available from Dynotech Laboratories Ltd). The read-out of the instrument is a 3 digit number in the range 0-999.
7. The reproducibility of the method has been assessed over a series of 35 assays, carried out by two operators, over a four week period. The inter-assay coefficient of variation (cv) for this series of experiments was 9.1%. It was found that if the assay had been performed correctly, the ratio of the positive control (lower reading) to the negative control (higher reading) was 0.612 ± 0.056 (i.e. ± 1 Standard Deviation (SD). For an "unknown" oil a value of 0.725 or greater indicated a "possibly counterfeit" sample.

Example 5

Assay of marked gasoline

13ml samples of leaded and unleaded gasoline were marked with 3 levels of PBA (10, 5 and 2.5 ppm). They were extracted with Tris buffer (2ml, 0.05M, pH 7.5). The extracts were assayed as described in Example 3 and PBA quantified using a calibration curve of standards prepared in an unmarked gasoline extract. Corresponding samples were prepared containing radiolabelled PBA enabling extraction efficiencies to be determined and allowing comparison between observed and expected values. The results are shown in Table 2.

TABLE 2

A comparison of observed and expected values for aqueous samples assayed against a calibration curve in unmarked gasoline extract

| Sample | Blank | 10ppm Extract | 10ppm Extract diluted 1:2 | 10ppm Extract diluted 1:4 | 10ppm Extract diluted 1:8 | 5ppm Extract | 5ppm Extract diluted 1:2 | 5ppm Extract diluted 1:4 | 2.5ppm Extract |
|---|---|---|---|---|---|---|---|---|---|
| Leaded gasoline | | | | | | | | | |
| Expected (radiochemistry) | 0 | 46* | 23 | 11.5 | 5.7 | 23 | 11.4 | 5.7 | 11.4 |
| Observed | 0.9 | 47 | 22.5 | 11.0 | 6.4 | 29 | 13.5 | 5.3 | 13.5 |
| Unleaded gasoline | | | | | | | | | |
| Expected (radiochemistry) | 0 | 33 | 16.2 | 8.1 | 4.1 | 15.5 | 7.7 | 3.9 | 7.5 |
| Observed | 0.9 | 41 | 18.5 | 7.0 | 3.8 | 14.5 | 6.2 | 3.4 | 7.6 |

* All values quoted in ppm.

These results demonstrate the concentration effect achieved by extracting PBA from 13ml of gasoline into 2ml of Tris buffer, and convincingly demonstrate that gasoline marked with PBA may be distinguished from unmarked gasoline by immunoassay.

Example 6

Assay of marked pharmaceuticals

Marked Samples of Paracetamol (acetaminophen - a mild pain killer and anti-pyretic agent) and Naproxen (a non-steroidal anti-inflammatory agent) were prepared containing 20ppm solid PBA. Portions of each (1g) were added to 10ml of PBS/Tween (described in Example 4) in a 20ml glass bottle. Similarly non-marked assay blanks were prepared for both materials. The bottles were tumbled overnight to extract the PBA into the PBS/Tween. Following separation of undissolved material by centrifugation, sample aliquots (250 µl) of the supernatant solutions were added to 250 µl of PBA specific antibody and analysed using an assay kit, similar to that described in Example 4.

From the results presented in Table 3 it was demonstrated that the marked pharmaceuticals were clearly distinguishable from the non-marked pharmaceuticals.

TABLE 3

| Drug | Unmarked* | Marked* | Ratio (%) |
|---|---|---|---|
| Paracetamol | 400 | 288 | 72 |
| | 394 | 275 | 70 |
| | 377 | 283 | 75 |
| | 378 | 278 | 74 |
| | 281 | 206 | 73 |
| | 266 | 203 | 76 |
| | 386 | 303 | 78 |
| | 398 | 267 | 67 |
| | 517 | 357 | 69 |
| | 480 | 378 | 79 |
| | 469 | 379 | 80 |
| | Mean Ratio=74 | S.D.=4.2 | C.V.=5.7% |
| Naproxen | 517 | 350 | 68 |
| | 503 | 337 | 67 |
| | 355 | 260 | 73 |
| | 313 | 226 | 72 |
| | 279 | 185 | 66 |
| | 269 | 220 | 81 |
| | 269 | 204 | 76 |
| | 245 | 153 | 64 |
| | Mean Ratio=71 | S.D.=5.7 | C.V=8.0% |

* Values indicated were read from the tube luminometer described in Example 4.

Example 7

Assay marked perfume

A sample of 'Eau de Cologne' was prepared containing 20ppm of PBA. 2ml of this marked material and 2ml of the unmarked material were placed in small glass test tubes and evaporated in a gentle air stream to incipient dryness. 2ml of PBS/Tween (described in Example 4) was added to the oily residue and the tube contents mixed vigorously by vortex mixing. The insoluble, heavier than water, oil was then separated by centrifugation and sample aliquots of the supernatant solution were analysed as described in Example 6. From the results presented in Table 3 it was demonstrated that the marked perfume was clearly distinguishable from the unmarked equivalent.

TABLE 4

| Perfume | Unmarked* | Marked* | Ratio (%) |
| --- | --- | --- | --- |
| Eau de Cologne | 866 | 104 | 12 |
| | 851 | 243 | 29 |
| | 655 | 107 | 16 |
| | 722 | 133 | 18 |
| | 710 | 126 | 18 |
| | 782 | 196 | 25 |
| | Mean Ratio=20 | S.D.=6.2 | C.V.=31.6% |

* Values indicated were read from the tube luminometer described in Example 4.

As an alternative approach marked and unmarked Eau de Cologne were diluted ten-fold with PBA/Tween and assayed directly as outlined in Example 6. The results are presented in Table 5 and show that this alternative method also permits clear distinction between unmarked and marked perfume.

TABLE 5

| Perfume | Unmarked* | Marked* | Ratio (%) |
| --- | --- | --- | --- |
| Eau de Cologne | 736 | 499 | 68 |
| | 760 | 522 | 69 |
| | 494 | 324 | 66 |
| | 382 | 267 | 70 |
| | 401 | 233 | 58 |
| | 385 | 257 | 68 |
| | 352 | 210 | 60 |
| | 484 | 307 | 63 |
| | 640 | 426 | 67 |
| | Mean Ratio=65.4 | S.D.=4.2 | C.V.=6.4% |

Example 8

Assay of marked drink

A sample of blended whisky was prepared containing 20ppm of PBA. The marked whisky and a corresponding sample of the unmarked whisky were diluted 4-fold with PBS/Tween and sample aliquots (250 μl) were analysed using the method described in Example 6. The results presented in Table 6 demonstrate that the marked whisky is clearly distinguishable from the non-marked whisky.

TABLE 6

| Drink | Unmarked* | Marked* | Ratio (%) |
|---|---|---|---|
| Whisky | 375 | 119 | 31 |
| | 396 | 158 | 39 |
| | 381 | 180 | 47 |
| | 449 | 110 | 25 |
| | 435 | 104 | 24 |
| | 427 | 109 | 26 |
| | 431 | 95 | 22 |
| | 421 | 116 | 28 |
| | 387 | 117 | 30 |
| | 440 | 86 | 19 |
| | 412 | 122 | 30 |
| | 386 | 122 | 32 |
| | 353 | 107 | 30 |
| | 474 | 115 | 24 |
| | 452 | 106 | 23 |
| | 446 | 111 | 25 |

Mean Ratio=28.4 S.D.=6.9 C.V.=24%

* Values indicated were obtained from the tube luminometer described in Example 4. It was noted in this Example that the signal generated took approximately 20 minutes to develop.

Note

The following terms used in the text are trade marks:
Amerlite
NH2 Bond Elut
Tween

## Claims

1. A method of authenticating a product comprising:
associating a marker compound with the product thereby to produce a "genuine" product, said marker compound, when associated with said product being visually undetectable; subsequently
providing from a test product whose authenticity it is desired to determine a sample, dissolved in aqueous medium, of any said marker compound associated with said test product;
identifying any said marker compound in said sample by an immunoassay specific for said marker; and
comparing the result of the immunoassay carried out on the sample from the test product with that obtainable when the same immunoassay is carried out on a sample in aqueous medium provided from said "genuine" product.

2. A method according to claim 1 wherein said marker compound in said sample provided from said test product is identified quantitatively.

3. A method according to claim 1 or claim 2 where the marker compound is mixed with the product.

4. A method according to any one of the previous claims wherein the product is an oil-based product, a perfume, a paint or a drink.

5. A method according to claim 4 wherein the product is an oil-based product and said marker compound has a log P in the range -2.5 to +5, where P is the partition coefficient of the marker compound between octanol and water at 25°C.

6. A method according to claim 3 wherein the product is a liquid oil-based product.

7. A method according to claim 6 wherein the product is a lubricating oil.

8. A method according to any one of the preceding claims wherein the marker compound is selected from aromatic carboxylic acids; phenols; ethers of polyhydric alcohols and phenols; amino acids; proteins; lipids; carbohydrates; and nucleic acids.

9. A method according to claim 8 wherein the marker compound is m-phenoxybenzoic acid.

10. A method according to claim 9 wherein the immunoassay employs monoclonal antibodies specific for m-phenoxybenzoic acid.

11. A method according to claim 9 wherein the immunoassay employs antibodies specific for a protein conjugate of m-phenoxybenzoic acid.

12. A method according to any of the preceding claims, wherein the marker compound is extracted from the product using a solvent comprising water and/or a water miscible organic solvent.

13. A method according to any one of the preceding claims wherein identification of the marker is carried out by competitive enzyme-linked immunosorbent assay.

**Patentansprüche**

1. Verfahren zum Bestimmen der Echtheit eines Produkts, umfassend:
Assoziieren einer Markerverbindung mit dem Produkt, um dadurch ein "echtes" Produkt herzustellen, wobei die mit dem Produkt assoziierte Markerverbindung visuell nicht bestimmbar ist; anschließend Bereitstellen einer Probe aus einem Testprodukt, dessen Echtheit bestimmt werden soll, die, gelöst in wäßrigem Medium, jegliche der mit dem Testprodukt assoziierten Markerverbindungen enthält;
Identifizieren jeder der Markerverbindungen in der Probe durch einen für den Marker spezifischen Immunoassay; und
Vergleichen des Ergebnisses des Immunoassays, der mit der Probe aus dem Testprodukt durchgeführt wurde, mit jenem, das erhalten wird, wenn derselbe Immunoassay mit einer Probe aus dem "echten" Produkt in wäßrigem Medium durchgeführt wird.

2. Verfahren nach Anspruch 1, worin die Markerverbindung in der Probe aus dem Testprodukt quantitativ bestimmt wird.

3. Verfahren nach Anspruch 1 oder 2, worin die Markerverbindung mit dem Produkt vermischt wird.

4. Verfahren nach irgendeinem der vorangegangenen Ansprüche, worin das Produkt ein Produkt auf Ölbasis, ein Parfum, ein Anstrichmittel oder ein Getränk ist.

5. Verfahren nach Anspruch 4, worin das Produkt ein Produkt auf Ölbasis ist und die Markerverbindung einen

logP im Bereich von -2,5 bis +5 besitzt, wobei P der Verteilungskoeffizient der Markerverbindung zwischen Octanol und Wasser bei 25°C ist.

6. Verfahren nach Anspruch 3, worin das Produkt ein flüssiges Produkt auf Ölbasis ist.

7. Verfahren nach Anspruch 6, worin das Produkt ein Schmieröl ist.

8. Verfahren nach irgendeinem der vorangegangenen Ansprüche, worin die Markerverbindung ausgewählt wird aus: aromatischen Carbonsäuren; Phenolen; Ethern von mehrwertigen Alkoholen und Phenolen; Aminosäuren; Proteinen; Lipiden; Kohlehydraten und Nukleinsäuren.

9. Verfahren nach Anspruch 8, worin die Markerverbindung m-Phenoxybenzoesäure ist.

10. Verfahren nach Anspruch 9, worin der Immunoassay monoklonale Antikörper verwendet, die für m-Phenoxybenzoesäure spezifisch sind.

11. Verfahren nach Anspruch 9, worin der Immunoassay Antikörper verwendet, die für ein Proteinkonjugat von m-Phenoxybenzoesäure spezifisch sind.

12. Verfahren nach irgendeinem der vorangegangenen Ansprüche, worin die Markerverbindung unter Verwendung eines Lösungsmittels, umfassend Wasser und/oder ein wassermischbares organisches Lösungsmittel, aus dem Produkt extrahiert wird.

13. Verfahren nach irgendeinem der vorangegangenen Ansprüche, worin die Identifizierung des Markers durch einen kompetitiven enzymgebundenen Immunosorbens-Assay erfolgt.

## Revendications

1. Méthode d'authentification d'un produit consistant à :
associer un composé marqueur au produit pour ainsi donner un produit "d'origine", ledit composé marqueur, quand il est associé audit produit étant visuellement indétectable; subséquemment.
se procurer, d'un produit de test dont on souhaite déterminer l'authenticité, un échantillon, dissous dans un milieu aqueux, de tout composé marqueur associé audit produit de test;
identifier tout composé marqueur dans ledit échantillon par un immunoessai spécifique dudit marqueur; et
comparer le résultat de l'immunoessai effectué sur l'échantillon du produit de test à celui pouvant être obtenu quand le même immunoessai est effectué sur un échantillon dans un milieu aqueux obtenu dudit produit "d'origine".

2. Méthode selon la revendication 1, où ledit composé marqueur dans ledit échantillon obtenu dudit produit de test est quantitativement identifié.

3. Méthode selon la revendication 1 ou la revendication 2 où le composé marqueur est mélangé au produit.

4. Méthode selon l'une quelconque des revendications précédentes où le produit est un produit à base d'huile, un parfum, une peinture ou une boisson.

5. Méthode selon la revendication 4 où le produit est un produit à base d'huile et ledit composé marqueur a un log P compris entre -2,5 et +5, P étant le coefficient de séparation du composé marqueur entre l'octanol et l'eau à 25°C.

6. Méthode selon la revendication 3 où le produit est un produit à base d'huile liquide.

7. Méthode selon la revendication 6 où ledit produit est une huile de lubrification.

8. Méthode selon l'une quelconque des revendications précédentes où le composé marqueur est choisi parmi des acides carboxyliques aromatiques; des phénols; des éthers d'alcools polyhydriques et phénols; des acides aminés; des protéines; des lipides; des carbohydrates; et des acides nucléiques.

**9.** Méthode selon la revendication 8 où ledit composé marqueur est l'acide m-phénoxybenzoïque.

**10.** Méthode selon la revendication 9 où l'immunoessai emploie des anticorps monoclonaux spécifiques de l'acide m-phénoxybenzoïque.

**11.** Méthode selon la revendication 9 où l'immunoessai emploie des anticorps spéficiques d'une protéine conjuguée de l'acide m-phénoxybenzoïque.

**12.** Méthode selon l'une quelconque des revendications précédentes où ledit marqueur est extrait du produit en utilisant un solvant comprenant de l'eau et/ou un solvant organique miscible dans l'eau.

**13.** Méthode selon l'une quelconque des revendications précédentes où l'identification du marqueur est effectuée par essai compétitif immosorbant lié à l'enzyme.